# NOUVEAU FASCICULE DE BREVET EUROPEEN

(11) **EP 0 859 581 B2**
(45) Date de publication et mention de la décision concernant l'opposition: **14.10.2009**
(45) Mention de la délivrance du brevet: 14.03.2001
(21) Numéro de dépôt: 96931852.6
(22) Date de dépôt: 16.09.1996
(51) Int. Cl.: A61K 8/81, A61K 8/31, A61K 8/893, A61Q 5/02, A61Q 5/06, A61Q 5/12, A61K 8/899

(54) **COMPOSITION POUR LE TRAITEMENT DES MATIERES KERATINIQUES COMPRENANT AU MOINS UN POLYMERE SILICONE GREFFE, A SQUELETTE POLYSILOXANIQUE GREFFE PAR DES MONOMERES ORGANIQUES NON-SILICONES ET AU MOINSUN HYDROCARBURE LIQUIDE EN C 11-C 26**
MITTEL ZUR BEHANDLUNG VON KERATINMATERIAL, DAS MINDESTENS EIN SILIKONGEPFROPFTES POLYMER MIT EINEM MIT NICHT-SILIKON ORGANISCHEN MONOMEREN GEPFROPFTEN POLYSILOXANGERÜST UND MINDESTENS EINEN FLÜSSIGEN C11-26 KOHLENWASSERSTOFF ENTHÄLT
COMPOSITION FOR TREATING KERATINOUS MATERIAL, INCLUDING AT LEAST ONE SILICONE-GRAFTED POLYMER WITH A POLYSILOXANE BACKBONE GRAFTED BY NON-SILICONE ORGANIC MONOMERS AND AT LEAST ONE C 11-26 LIQUID HYDROCARBON

(30) Priorité: 29.09.1995 FR 9511480
(43) Date de publication de la demande: 26.08.1998
(73) Titulaire: L'ORÉAL, 75008 Paris (FR)
(72) Inventeur: DUBIEF, Claude, F-78150 Le Chesnay (FR); DUPUIS, Christine, F-75018 Paris (FR); CAUWET-MARTIN, Danièle, F-75011 Paris (FR)
(74) Mandataire: Dossmann, Gérard
(86) Numéro de dépôt international: PCT/FR1996/001434
(87) Numéro de publication internationale: WO 1997/012585

(56) Documents cités:
- EP-A- 0 388 582
- EP-A- 0 636 361
- EP-A1- 0 498 716
- EP-A2- 0 035 899
- EP-A2- 0 240 350
- EP-A2- 0 679 386
- EP-B1- 0 697 208
- WO-A-93/23446
- WO-A-95/03776
- WO-A-95/04518
- WO-A-95/05800
- WO-A-95/06078
- WO-A1-93//05762
- WO-A1-95//06078
- WO-A1-95//25503
- WO-A1-97//01321
- DE-C1- 4 241 799
- DE-C1- 4 426 794
- US- - 3 577 528
- US- - 3 818 105
- 'Cosmetic Raw Material Analysis and Quality', 1994, INTERNATIONAL FEDERATION OF THE SOCIETIES OF COSMETIC CHEMIST BY MICELLE PRESS, ENGLAND, ISBN 1870228111 article LUIGI RIGANO AND TASUKU TAKAMATSU, pages 27 - 27
- DATENBLATT ZU ISOPAR, VON ESSO CHEMIE
- DATENBLATT ZU DRAKEOL 7 MINERAL OIL VON PENRECO

## Description

La présente invention a trait à une composition cosmétique ou dermatologique pour le traitement des cheveux, en particulier des cheveux humains, comprenant au moins un polymère siliconé greffé, à squelette polysiloxanique greffé par des monomères organiques non-siliconés et au moins un hydrocarbure liquide à température ambiante dont la chaîne hydrocarbonée présente de 11 à 26 atomes de carbone ainsi que ses applications notamment dans le domaine de la cosmétique capillaire.

Les polymères du type polymère à squelette polysiloxanique greffé par des monomères organiques non-silicones sont connus pour leurs propriétés coiffantes. Ces polymères conduisent cependant à des propriétés cosmétiques insuffisantes après application.

La demanderesse a trouvé de façon surprenante qu'en associant à ces types de polymère des hydrocarbures linéaires ou ramifiés, cycliques ou acycliques, liquides à température ambiante, dont la chaîne hydrocarbonée présente de 11 à 26 atomes de carbone, on améliorait les propriétés cosmétiques notamment le démêlage des cheveux ainsi que la douceur au toucher tout en conservant les propriétés coiffantes de ces polymères.

La composition cosmétique ou dermatologique de traitement des cheveux est caractérisée par le fait qu'elle contient dans un milieu cosmétiquement ou dermatologiquement acceptable constitué par de l'eau ou un mélange d'eau et d'au moins un solvant cosmétiquement acceptable et/ou étant conditionnée sous forme de vaporisateur, de flacon-pompe ou dans un récipient aérosol en vue d'obtenir un spray, une laque ou une mousse ladite composition contenant : au moins un polymère siliconé greffé, à squelette polysiloxanique greffé par des monomères organiques non-siliconés susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique choisi, seul ou en mélanges de monomères, parmi les esters d'acide acrylique d'alcanol et/ou les esters d'acide methacrylique d'alcanol, de préférence l'alcanol étant en C₁-C₁₈ et d'autre part un polysiloxane présentant dans sa chaîne au moins un, et de préférence plusieurs, groupements fonctionnels capables de venir réagir sur lesdites insaturations éthyléniques desdits monomères non-siliconés et au moins un hydrocarbure liquide à température ambiante dont la chaîne hydrocarbonée présente de 11 à 26 atomes de carbone, linéaire ou ramifié, cyclique ou acyclique.

Dans ce qui suit, on entend désigner par polymère siliconé, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxys, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxy-alkylamino ou aminoalkyls, des groupements ammonium quatemaire, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés greffés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A-0582152, WO93/23009 et WO95/03776 dont les enseignements sont totalement inclus dans la présente description à titre de références non limitatives.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléfique, l'acide itaconique, l'acide fumarique et l'acide crotonique. Les sels convenables sont notamment les sels d'alcalins, d'alcalino-terreux et d'ammonium. On notera que, de même, dans le polymère silicone greffé final, le groupement organique à caractère anionique qui comprend le résultat de l'(homo) polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle ou leurs mélanges.

Une famille de polymères silicones greffés particulièrement bien à la mise en oeuvre de la présente invention est constituée par les polymères silicones comportant dans leur structure le motif de formule (I) suivant 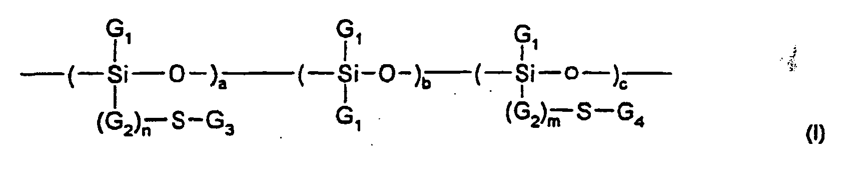 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle (C₁-C₁₀) de préférence le (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Le polymère siliconé greffé est utilisé de préférence en une quantité allant de 0,01 à 20% en poids du poids total de la composition. Plus préférentiellement, cette quantité varie de 0,1 à 15% en poids et encore plus préférentiellement de 0,5 à 10% en poids.

Les hydrocarbures linéaires ou ramifiés, cycliques ou acycliques, liquides à température ambiante, conformes à l'invention, sont choisis de préférence parmi l'isododécane (C₁₂), l'isohexadecane(C₁₆) et ses isomères tels que le 2,2,4,4,6,6-heptaméthylnonane (C₁₆), l'isoeicosane (C₂₀), l'isotétracosane (C₂₄) et leurs isomères. On utilise plus particulièrement l'isododécane (C₁₂) ou l'un de ses isomères.

Les hydrocarbures liquides conformes à l'invention sont utilisés de préférence en une quantité allant de 0,01 à 10% en poids du poids total de la composition. Encore plus préférentiellement, cette quantité varie de 0,5 à 5% en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit mileu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé non-thérapeutique de traitement des matières kératiniques telles que les cheveux consistant à appliquer sur celles-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLES

### EXEMPLE 1 Spray de coiffage en flacon pompe

- Polymère siliconé greffé de formule (I) de structure polyméthyl/méthylsiloxane à groupements propyl thio-3 acide polyméthacrylique et groupements propyl thio-3 polyméthacrylate de méthyle 6 g en MA
- Isododécane (C₁₂) 3 g
- Aminométhylpropanol neutralisation à 100% dudit polymère siliconé qsp
- Ethanol qsp 100 g

### EXEMPLE 2 Spray de coiffage en aérosol

- Polymère siliconé greffé de formule (I) de structure polyméthyl/méthylsiloxane à groupements propyl thio-3 acide polyméthacrylique et groupements propyl thio-3 polyméthacrylate de méthyle 5 g en MA
- Copolymère acétate de vinyle/acide crotonique/tertio-butyl-4-benzoate de vinyle (65/10/25) neutralisé tel que décrit et préparé dans le brevet FR 2.697.160 2,5 g en MA
- isododécane (C₁₂) vendu sous le nom PERMETHYIL99A par la société PRESPERSE INC. 3 g
- Aminométhylpropanol neutralisation à 100% dudit polymère siliconé greffé et du copolymèr qsp
- Ethanol qsp 100 g

### Schéma de pressurisation :

- Composition ci-dessus 80 g
- Isobutane 15 g
- 1,1-difluoroéthane 5 g

### EXEMPLE 3 Gel de coiffage

- Polymère silicone greffé de formule (I) de structure polyméthyl/méthylsiloxane à groupements propyl thio-3 acide polyméthacrylique et groupements propyl thio-3 polyméthacrylate de méthyle 4 g en MA
- Polymère poly(acide acrylique) vendu sous le nom SYNTHALEN K par la société 3 V 0,5 g en MA
- Copolymère vinylpyrrolidone/acétate de vinyle vendu sous le nom PVPNA 735 par la société ISP 1 g en MA
- 2,2,4,4,6,6 heptaméthylnonane (C₁₆) vendu sous le nom ARLANOL HD par ICI 3 g
- Aminométhylpropanol neutralisation à 100% dudit polymère siliconé et du polymère acrylique qsp
- Ethanol 20 g
- Eau déminéralisée qsp 100 g

### EXEMPLE 4 Shampooing

- Polymère siliconé greffé de formule (I) de structure polyméthyl/méthylsiloxane groupements propyl thio-3 polyméthacrylate d'isobutyle 1 g en MA
- Isoeicosane (C₂₀) 2 g
- Lauryl éther sulfate de sodium (C₁₂/C₁₄ ; 70/30) à 22 moles d'oxyde d'éthylène en solution aqueuse vendu sous le nom EMPICOL ESB 31/F par la société ALBRIGLET et WILSON 15 g
- Cocoylbétaïne 3 g
- Cétostéaryl sulfate de sodium (C₁₆/C₁₈ ; 50/50) vendu sous le nom de LANETH par HENHEL 0,8 g
- Mélange 1-(hexadécyloxy)-2 octadécanol/alcool cétylique (47/53) 2,5 g
- Monoisopropanolamide d'acide de coprah 2 g
- Parfum, conservateur qs
- Eau qsp pH ajusté à 5,5 par HCl 100 g

### EXEMPLE 5 Après-shampooing

- Polymère siliconé greffé de formule (I) de structure polyméthyl/méthylsiloxane groupements propyl thio-3 polyméthacrylate d'isobutyle 1 g en MA
- 2,2,4,4,6,6 heptaméthylnonane (C₁₆) vendu sous le nom ARLANOL HD par ICI 2 g
- Copolymère acrylamide/acide acrylamido 2- méthylpropane sulfonique sous forme de sel de sodium en émulsion inverse à 40% dans un mélange isoparaffine/eau tel que le produit décrit dans l'exemple 1 du document EP-A-503 853 1 g en MA
- Mélange polydiméthylsiloxane α,ω-dihydroxylé /cyclotétra- et cyclopentadiméthylsiloxane (56/44) (14/86) vendu sous le nom QCF2-1671 FLUID par DOW CORNING 10 g
- Parfum, conservateur qs
- Eau qsp pH ajusté à 6 par NaOH 100 g

## Revendications

1. Composition cosmétique ou dermatologique de traitement des cheveux, **caractérisée par le fait qu'**elle contient dans un milieu cosmétiquement ou dermatologiquement acceptable constitué par de l'eau ou un mélange d'eau et d'au moins un solvant cosmétiquement acceptable et/ou étant conditionnée sous forme de vaporisateur, de flacon pompe ou dans un récipient aérosol en vue d'obtenir un spray, une laque ou une mousse ladite composition contenant au moins un polymère siliconé greffé, à squelette polysiloxanique greffé par des monomères organiques non-siliconés susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique choisi, seul ou en mélanges de monomères, parmi les esters d'acide acrylique d'alcanol et/ou les esters d'acide methacrylique d'alcanol, de préférence l'alcanol étant en C₁-C₁₈ et d'autre part un polysiloxane présentant dans sa chaîne au moins un, et de préférence plusieurs, groupements fonctionnels capables de venir réagir sur lesdites insaturations éthyléniques desdits monomères non-siliconés et au moins un hydrocarbure liquide à température ambiante dont la chaîne hydrocarbonée présente de 11 à 26 atomes de carbone, linéaire ou ramifié, cyclique ou acyclique.

2. Composition selon la revendication 1, **caractérisée par le fait que** le monomères organique anionique à insaturation éthylénique est choisi, seul ou sous forme de mélange de monomères, parmi les acides carboxyliques insaturés, linéaires ou ramifiés.

3. Composition selon la revendication 2, **caractérisée par le fait que** le monomères organique anionique à insaturation éthylénique est choisi, seul ou sous forme de mélange de monomères, parmi l'acide acrylique, l'acide méthacrylique, l'acide maléfique l'anhydride maléique, l'acide itaconique, l'acide fumarique et l'acide crotonique ou leurs sels d'alcalins, d'alcalino-terreux ou d'ammonium, ou leurs mélanges.

4. Composition selon la revendication 1, **caractérisée par le fait que** le monomère organique hydrophobe à insaturation éthylénique est choisi, seul ou en mélange de monomères dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle.

5. Composition selon l'une quelconque des revendications 1 à 5, **caractérisée par le fait que** le polymère siliconé greffé comprend sur la chaîne silicone principale, au moins un groupement organique à caractère anionique obtenu par l'(homo) polymérisation radicalaire d'au moins un monomère anionique de type acide carboxylique insaturé partiellement ou totalement neutralisé sous la forme d'un sel.

6. Composition selon l'une quelconque des revendications 1 à 5, **caractérisée par le fait que** le polymère siliconé greffé est choisi parmi les polymères siliconés comportant dans leur structure le motif de formule (I) suivant : 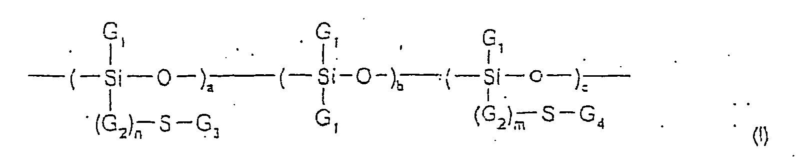 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

7. Composition selon la revendication 6, **caractérisée par le fait que** le motif de formule (I) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle (C₁-C₁₀).

8. Composition selon la revendication 6 ou 7, **caractérisée par le fait que** le motif de formule (I) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobutyle ou de méthyle.

9. Composition selon l'une quelconque des revendications 1 à 8, **caractérisée par le fait que** la masse moléculaire en nombre du polymère siliconé greffé varie de 10 000 à 1 000 000 environ, et encore plus préférentiellement de 10 000 à 100 000 environ.

10. Composition selon l'une quelconque des revendications 1 à 9, **caractérisée par le fait que** le polymère siliconé greffé est utilisé en une quantité allant de 0,01 à 20% en poids par rapport au poids total de la composition et de préférence de 0,5 à 15% en poids et préférentiellement, cette quantité varie de 0,1 à 15% en poids et encore plus préférentiellement de 0,5 à 10% en poids.

11. Composition selon l'une quelconque des revendications 1 à 10, **caractérisée par le fait que** les hydrocarbures linéaires ou ramifiés, cycliques ou acycliques, liquides à température ambiante, sont choisis dans le groupe constitué par l'isododécane (C₁₂), l'isohexadecane (C₁₆), l'isoeicosane (C₂₀), l'isotétracosane (C₂₄) et leurs isomères.

12. Composition selon l'une quelconque des revendications 1 à 11, **caractérisée par le fait qu'**elle contient l'isododécane (C₁₂) ou l'un de ses isomères.

13. Composition selon l'une quelconque des revendications 1 à 15, **caractérisée par le fait que** les hydrocarbures liquides en C₁₁-C₂₅ sont utilisés en une quantité comprise allant de 0,01 à 10% en poids du poids total de la composition et plus préférentiellement de 0,5 à 5% en poids.

14. Composition selon l'une quelconque des revendications 1 à 16, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

15. Composition selon l'une des revendications précédentes, **caractérisée par le fait que** les solvants cosmétiquement acceptables sont choisis dans le groupe constitué par les monoalcools, les polyalcools, les éthers de glycol, les esters d'acides gras et leurs mélanges.

16. Composition selon l'une quelconque des revendications 1 à 15, **caractérisée par le fait que** le polymère siliconé greffé est dissous dans le milieu cosmétiquement ou dermatologiquement acceptable ou utilisé sous forme de dispersion aqueuse de particules.

17. Composition selon l'une quelconque des revendications 1 à 16, **caractérisée par le fait que** les matières kératiniques sont des cheveux humains.

18. Composition selon l'une quelconque des revendications 1 à 17, **caractérisée par le fait qu'**elle se présente sous forme de gel, de lait, de crème, de lotion plus ou moins épaissie ou de mousse.

19. Composition selon l'une quelconque des revendications 1 à 18, **caractérisée par le fait qu'**elle est un produit de coffrage.

20. Composition selon l'une quelconque des revendications 1 à 19, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

21. Procédé non-thérapeutique de traitement des matières kératiniques, en particulier des cheveux humains, **caractérisé par le fait qu'**il consiste à appliquer sur lesdites matières une composition telle que définie selon l'une quelconque des revendications 1 à 20 puis à effectuer éventuellement un rinçage à l'eau.

## Claims

1. Cosmetic or dermatological composition for the treatment of the hair, **characterized in that** it contains, in a cosmetically or dermatologically acceptable medium constituted of water or of a mixture of water and at least one cosmetically acceptable solvent, and/or is packaged in the form of a vaporizer or a pump-dispenser bottle or alternatively in an aerosol can in order to obtain a spray, a lacquer or a foam, the said composition containing at least one grafted silicone polymer, containing a polysiloxane skeleton grafted with non-silicone organic monomers, which can be obtained by radical copolymerization between, on the one hand, at least one non-silicone anionic organic monomer having ethylenic unsaturation and/or a non-silicone hydrophobic organic monomer having ethylenic unsaturation which is chosen, alone or as mixtures of monomers, from acrylic acid esters of an alkanol and/or methacrylic acid esters of an alkanol, the alkanol preferably being C₁-C₁₈, and, on the other hand, a polysiloxane having in its chain at least one, and preferably several, functional group(s) capable of reacting with the said ethylenic unsaturations of the said non-silicone monomers and at least one hydrocarbon which is liquid at room temperature, the hydrocarbon chain of which has from 11 to 26 linear or branched, cyclic or acyclic carbon atoms.

2. Composition according to Claim 1, **characterized in that** the anionic organic monomer containing ethylenic unsaturation is chosen, alone or in the form of a mixture of monomers, from linear or branched, unsaturated carboxylic acids.

3. Composition according to Claim 2, **characterized in that** the anionic organic monomer containing ethylenic unsaturation is chosen, alone or in the form of a mixture of monomers, from acrylic acid, methacrylic acid, maleic acid, maleic anhydride, itaconic acid, fumaric acid and crotonic acid, or alkali metal, alkaline-earth metal or ammonium salts thereof, or mixtures thereof.

4. Composition according to Claim 1, **characterized in that** the hydrophobic organic monomer containing ethylenic unsaturation is chosen, alone or as a mixture of monomers, from the group constituted of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

5. Composition according to any one of Claims 1 to 4, **characterized in that** the grafted silicone polymer comprises, on the main silicone chain, at least one organic group of anionic nature obtained by radical (homo)polymerization of at least one anionic monomer of unsaturated carboxylic acid type, partially or totally neutralized in the form of a salt.

6. Composition according to any one of Claims 1 to 5, **characterized in that** the grafted silicone polymer is chosen from silicone polymers containing in their structure the unit of formula (I) below: 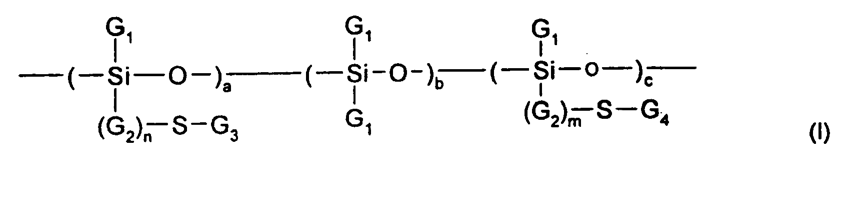 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350; c is an integer ranging from 0 to 50; with the proviso that one of the parameters a and c is other than 0.

7. Composition according to Claim 6, **characterized in that** the unit of formula (I) has at least one of the following characteristics:
- the radicals G₁ denote a C₁-C₁₀ alkyl radical;
- n is non-zero, and the radicals G₂ represent a divalent C₁-C₃ radical;
- G₃ represents a polymer radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

8. Composition according to Claim 6 or 7, **characterized in that** the unit of formula (I) simultaneously has the following characteristics:
- the radicals G₁ denote a methyl radical;
- n is non-zero and the radicals G₂ represent a propylene radical;
- G₃ represents a polymer radical resulting from the (homo)polymerization of at least acrylic acid and/or methacrylic acid;
- G₄ represents a polymer radical resulting from the (homo)polymerization of at least one monomer of the isobutyl or methyl (meth)acrylate type.

9. Composition according to any one of Claims 1 to 8, **characterized in that** the number-average molecular mass of the grafted silicone polymer ranges approximately from 10,000 to 1,000,000 and even more preferably approximately from 10,000 to 100,000.

10. Composition according to any one of Claims 1 to 9, **characterized in that** the grafted silicone polymer is used in an amount ranging from 0.01 to 20% by weight relative to the total weight of the composition and preferably from 0.5 to 15% by weight and, preferably, this amount ranges from 0.1 to 15% by weight and even more preferably from 0.5 to 10% by weight.

11. Composition according to any one of Claims 1 to 10, **characterized in that** the linear or branched, cyclic or acyclic hydrocarbons that are liquid at room temperature are chosen from the group constituted of isododecane (C₁₂), isohexadecane (C₁₆), isoeicosane (C₂₀) and isotetracosane (C₂₄) and isomers thereof.

12. Composition according to any one of Claims 1 to 11, **characterized in that** it contains isododecane (C₁₂) or one of the isomers thereof.

13. Composition according to any one of Claims 1 to 12, **characterized in that** the C₁₁-C₂₅ liquid hydrocarbons are used in an amount ranging from 0.01 to 10% by weight of the total weight of the composition, and more preferably from 0.5 to 5% by weight.

14. Composition according to any one of Claims 1 to 13, **characterized in that** it also contains at least one additive chosen from the group constituted of thickeners, fatty acid esters, fatty acid esters of glycerol, silicones, surfactants, fragrances, preserving agents, sunscreens, proteins, vitamins, polymers, plant, animal, mineral or synthetic oils and any other additive conventionally used in the cosmetics field.

15. Composition according to one of the preceding claims, **characterized in that** the cosmetically acceptable solvents are chosen from the group constituted of monoalcohols, polyalcohols, glycol ethers and fatty acid esters, and mixtures thereof.

16. Composition according to any one of Claims 1 to 15, **characterized in that** the grafted silicone polymer is dissolved in the cosmetically or dermatologically acceptable medium or used in the form of an aqueous dispersion of particles.

17. Composition according to any one of Claims 1 to 16, **characterized in that** the keratinous material is human hair.

18. Composition according to any one of Claims 1 to 17, **characterized in that** it is in the form of a gel, a milk, a cream, a more or less thickened lotion or a foam.

19. Composition according to any one of Claims 1 to 18, **characterized in that** it is a hairstyling product.

20. Composition according to any one of Claims 1 to 19, **characterized in that** it is a hair product chosen from the group constituted of shampoos and rinse-out or leave-in hair products to be applied before or after shampooing, dyeing, bleaching, permanent-waving or straightening of the hair.

21. Non-therapeutic process for the treatment of keratinous material, in particular human hair, **characterized in that** it consists in applying to the said material a composition as defined according to any one of Claims 1 to 20, optionally followed by rinsing with water.

## Patentansprüche

1. Kosmetische oder dermatologische Zusammensetzung zur Behandlung der Haare, **dadurch gekennzeichnet, dass** sie in einem kosmetisch oder dermatologisch akzeptablen Medium, das aus Wasser oder einem Gemisch von Wasser und mindestens einem kosmetisch akzeptablen Lösungsmittel besteht und/oder in Form eines Zerstäubers, Pumpflakons oder Aerosolbehälters konfektioniert ist, um ein Spray, einen Lack oder einen Schaum zu bilden, mindestens ein gepfropftes Siliconpolymer mit Polysiloxangerüst, auf das nicht siliconhaltige organische Monomere gepfropft sind, das durch radikalische Copolymerisation einerseits mindestens eines nicht siliconhaltigen, anionischen, organischen Monomers mit ethylenisch ungesättigter Bindung und/oder eines nicht siliconhaltigen, hydrophoben, organischen Monomers mit ethylenisch ungesättigter Bindung, das einzeln oder in Form von Monomerengemischen unter den Estern von Acrylsäure und einem Alkanol und/oder den Estern von Methacrylsäure und einem Alkanol ausgewählt ist, wobei das Alkanol vorzugsweise 1 bis 18 Kohlenstoffatome aufweist, und andererseits eines Polysiloxans erhältlich ist, das in seiner Kette mindestens eine und vorzugsweise mehrere funktionelle Gruppen aufweist, die befähigt sind, mit den ethylenisch ungesättigten Bindungen der nicht siliconhaltigen Monomere zu reagieren, und mindestens einen geradkettigen oder verzweigten, cyclischen oder acyclischen, bei Raumtemperatur flüssigen Kohlenwasserstoff enthält, dessen Kohlenwasserstoffkette 11 bis 26 Kohlenstoffatome aufweist.

2. Zusammensetzung nach Anspruch 1, **dadurch gekennzeichnet, dass** das anionische organische Monomer mit ethylenisch ungesättigter Bindung einzeln oder in Form von Monomerengemischen unter den geradkettigen oder verzweigten, ungesättigten Carbonsäuren ausgewählt ist.

3. Zusammensetzung nach Anspruch 2, **dadurch gekennzeichnet, dass** das anionische organische Monomer mit ethylenisch ungesättigter Bindung einzeln oder in Form eines Gemisches von Monomeren unter Acrylsäure, Methacrylsäure, Maleinsäure, Maleinsäureanhydrid, Itaconsäure, Fumarsäure, Crotonsäure oder deren Alkali-, Erdalkali- oder Ammoniumsalzen oder deren Gemischen ausgewählt ist.

4. Zusammensetzung nach Anspruch 1, **dadurch gekennzeichnet, dass** das hydrophobe organische Monomer mit ethylenisch ungesättigter Bindung einzeln oder in Form von Monomerengemischen unter Isooctyl(meth)acrylat, Isononyl(meth)acrylat, 2-Ethylhexyl(meth)acrylat, Lauryl(meth)acrylat, Isopentyl(meth)-acrylat, n-Butyl(meth)acrylat, Isobutyl(meth)acrylat, Methyl-(meth)acrylat, t-Butyl(meth)acrylat, Tridecyl(meth)acrylat und Stearyl(meth)acrylat ausgewählt ist.

5. Zusammensetzung nach einem der Ansprüche 1 bis 4, **dadurch gekennzeichnet, dass** das gepfropfte Siliconpolymer in der Siliconhauptkette mindestens eine organische Gruppe mit anionischen Eigenschaften aufweist, die durch radikalische (Homo)-polymerisation mindestens eines anionischen Monomers vom Typ einer ungesättigten, zum Teil oder vollständig in Form eines Salzes neutralisierten Carbonsäure erhalten wird.

6. Zusammensetzung nach einem der Ansprüche 1 bis 5, **dadurch gekennzeichnet, dass** das gepfropfte Siliconpolymer unter den Siliconpolymeren ausgewählt ist, die in ihrer Struktur die Einheit der folgenden Formel (I) enthalten: 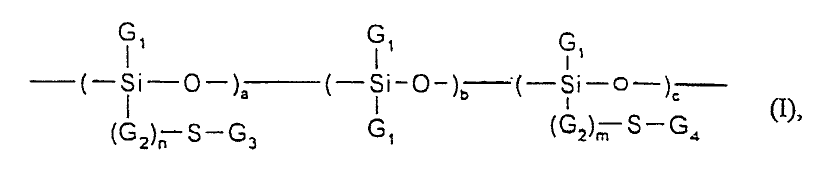 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenisch ungesättigter Bindung resultiert; G₄ eine Polymergruppe, die aus der (Homo)polymerisation mindestens eines hydrophoben Monomers mit ethylenisch ungesättigter Bindung resultiert; m und n 0 oder 1; a 0 oder eine ganze Zahl im Bereich von 1 bis 50; b eine ganze Zahl im Bereich von 10 bis 350 und c 0 oder eine ganze Zahl von 1 bis 50 bedeuten, mit der Maßgabe, dass einer der Parameter a oder c von 0 verschieden ist.

7. Zusammensetzung nach Anspruch 6, **dadurch gekennzeichnet, dass** die Einheit der Formel (I) mindestens eines der folgenden Merkmale aufweist:
· die Gruppen G₁ bedeuten eine C₁₋₁₀-Alkylgruppe;
· n ist nicht 0 und die Gruppen G₂ bedeuten eine zweiwertige Gruppe mit 1 bis 3 Kohlenstoffatomen;
· G₃ bedeutet eine Polymergruppe, die aus der (Homo)polymerisation mindestens eines Monomers vom Typ einer Carbonsäure mit ethylenisch ungesättigter Bindung resultiert;
· G₄ bedeutet eine Polymergruppe, die aus der (Homo)polymerisation mindestens eines Monomers vom Typ C₁₋₁₀-Alkyl(meth)acrylat resultiert.

8. Zusammensetzung nach Anspruch 6 oder 7, **dadurch gekennzeichnet, dass** die Einheit der Formel (I) alle folgenden Merkmale aufweist:
· die Gruppen G₁ bedeuten Methyl;
· n ist nicht 0 und die Gruppen G₂ bedeuten Propylen;
· G₃ bedeutet eine Polymergruppe, die aus der (Homo)polymerisation von zumindest Acrylsäure und/ oder Methacrylsäure resultiert;
· G₄ bedeutet eine Polymergruppe, die aus der (Homo)polymerisation mindestens eines Monomers vom Typ Isobutyl(meth)acrylat oder Methyl(meth)acrylat resultiert.

9. Zusammensetzung nach einem der Ansprüche 1 bis 8, **dadurch gekennzeichnet, dass** die zahlenmittlere Molmasse des gepfropften Siliconpolymers im Bereich von etwa 10 000 bis 1 000 000 und noch bevorzugter von etwa 10 000 bis 100 000 liegt.

10. Zusammensetzung nach einem der Ansprüche 1 bis 9, **dadurch gekennzeichnet, dass** das gepfropfte Siliconpolymer in einer Menge von 0,01 bis 20 Gew.-%, bezogen auf das Gesamtgewicht der Zusammensetzung, und vorzugsweise 0,5 bis 15 Gew.-% verwendet wird, wobei der Mengenanteil noch bevorzugter im Bereich von 0,1 bis 15 Gew.-% und besonders bevorzugt im Bereich von 0,5 bis 10 Gew.-% liegt.

11. Zusammensetzung nach einem der Ansprüche 1 bis 10, **dadurch gekennzeichnet, dass** die geradkettigen oder verzweigten, cyclischen oder acyclischen, bei Raumtemperatur flüssigen Kohlenwasserstoffe unter Isododecan (C₁₂), Isohexadecan (C₁₆), Isoeicosan (C₂₀), Isotetracosan (C₂₄) und deren Isomeren ausgewählt sind.

12. Zusammensetzung nach einem der Ansprüche 1 bis 11, **dadurch gekennzeichnet, dass** sie Isododecan (C₁₂) oder eines seiner Isomere enthält.

13. Zusammensetzung nach einem der Ansprüche 1 bis 15, **dadurch gekennzeichnet, dass** die flüssigen C₁₁-₂₆-Kohlenwasserstoffe in einer Menge von 0,01 bis 10 Gew.-% des Gesamtgewichts der Zusammensetzung und vorzugsweise 0,5 bis 5 Gew.-% verwendet werden.

14. Zusammensetzung nach einem der Ansprüche 1 bis 16, **dadurch gekennzeichnet, dass** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

15. Zusammensetzung nach einem der vorhergehenden Ansprüche, **dadurch gekennzeichnet, dass** die kosmetisch akzeptablen Lösungsmittel unter Monoalkoholen, Polyalkoholen, Glycolethern, Fettsäureestern und deren Gemischen ausgewählt sind.

16. Zusammensetzung nach einem der Ansprüche 1 bis 15, **dadurch gekennzeichnet, dass** das gepfropfte Siliconpolymer in dem kosmetisch oder dermatologisch akzeptablen Medium gelöst ist oder in Form einer wässrigen Dispersion von Partikeln verwendet wird.

17. Zusammensetzung nach einem der Ansprüche 1 bis 16, **dadurch gekennzeichnet, dass** es sich bei den Keratinsubstanzen um menschliches Haar handelt.

18. Zusammensetzung nach einem der Ansprüche 1 bis 17, **dadurch gekennzeichnet, dass** sie als Gel, Milch, Creme, mehr oder weniger dickflüssige Lotion oder Schaum vorliegt.

19. Zusammensetzung nach einem der Ansprüche 1 bis 18, **dadurch gekennzeichnet, dass** es sich um ein Produkt für die Frisurengestaltung handelt.

20. Zusammensetzung nach einem der Ansprüche 1 bis 19, **dadurch gekennzeichnet, dass** es sich um ein Produkt für das Haar handelt, das unter den Haarwaschmitteln und Produkten für das Haar, die ausgespült oder nicht ausgespült werden, vor oder nach einer Haarwäsche, Färbung, Entfärbung, Dauerwelle oder Entkräuselung aufgetragen werden, ausgewählt ist.

21. Nicht therapeutisches Verfahren zur Behandlung von Keratinsubstanzen und insbesondere menschlichem Haar, **dadurch gekennzeichnet, dass** es darin besteht, auf die Keratinsubstanzen eine Zusammensetzung nach einem der Ansprüche 1 bis 20 auf zutragen und anschließend gegebenenfalls mit Wasser zu spülen.
